(19) Europäisches Patentamt
European Patent Office
Office européen des brevets

(11) EP 1 262 204 A1

(12) **EUROPEAN PATENT APPLICATION**
published in accordance with Art. 158(3) EPC

(43) Date of publication:
**04.12.2002 Bulletin 2002/49**

(21) Application number: **01912245.6**

(22) Date of filing: **09.03.2001**

(51) Int Cl.[7]: **A61M 1/34**, B01D 39/16

(86) International application number:
**PCT/JP01/01880**

(87) International publication number:
**WO 01/066171 (13.09.2001 Gazette 2001/37)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **10.03.2000 JP 2000066683**

(71) Applicant: **ASAHI MEDICAL Co., Ltd.**
**Tokyo 101-8482 (JP)**

(72) Inventor: **HAYASHI, Shizue**
**Kawasaki-shi, Kanagawa 211-0018 (JP)**

(74) Representative:
**von Kreisler, Alek, Dipl.-Chem. et al**
**Patentanwälte**
**von Kreisler-Selting-Werner**
**Postfach 10 22 41**
**50462 Köln (DE)**

# (54) NOVEL LEUKAPHERETIC FILTER

(57) A filter medium with which a leukocyte-containing fluid represented by whole blood can be treated to selectively remove the leukocytes therefrom and recover the erythrocytes, thrombocytes, and blood plasma by allowing these to pass through the medium.

The leukocyte-removing filter is characterized by comprising a polymer having hydrophobic structural units and hydrophilic structural units and a porous substrate.

The polymer is, for example, a copolymer of a hydrophobic monomer and a hydrophilic monomer or a hydrophilic polymer having hydrophobic structural units introduced therein by modification or chemical modification.

EP 1 262 204 A1

## Description

FIELD OF THE INVENTION

The present invention relates to a blood filter for efficiently removing leukocytes. More particularly, the present invention relates to a leukocyte-removing filter that can selectively remove only leukocytes from leukocyte-containing fluid such as whole blood by easily filtering out erythrocytes, blood plasma, and thrombocytes.

BACKGROUND ART

Leukocyte-removing technology has been attracting attention as a most important subject in recent blood transfusion technology. A number of researchers have carried out for the purpose of reducing the physical burden on the patients after transfusion. For example, in order to prevent side effects and communicable diseases induced by leukocytes as a major causative substance, such as induction of graft versus host disease (GVHD), side effects due to anhemolytic fever, production of anti-leukocyte antibodies, and infection induced by viral-infected leukocytes, leukocytes have been removed or inactivated by centrifugation, filtration or radiation from blood products for transfusion. In particular, leukocyte reduction by filtration is widely accepted as an effective method simply and easily available at the bedside due to its simplicity and low cost.

Another important advantage of leukocyte reduction is in the improvement of storage stability and safety of blood products used for blood component transfusion. Specifically, when blood products containing leukocytes are stored for a long time, it becomes difficult to prevent pyrogenic cytokines from being produced by leukocytes during storage, further it also becomes extremely difficult to prevent adverse effects such as dispersion of pathogenetic media produced by death or crushing of leukocytes holding viruses and bacteria into the blood preparation. For these reasons, necessity of removing as many leukocytes in the blood preparation as possible before storing has been pointed out. Development of an effective and aseptic leukocyte reduction technology has also been strongly desired in this point (T. Asai, K. Hiruma, Y. Hoshi, "BLOOD TRANSFUSION UNDERSTOOD AT A GLANCE", page 77, published by Medical Science International, Inc.).

However, conventional leukocyte reduction technologies have required complicated operations of fractionating blood (whole blood) collected from donors into various components by centrifugation and then removing leukocytes from each blood preparation obtained by the fractionation. Furthermore, in addition to the complicated procedures and cost involved in separation and purification, conventional leukocyte reduction methods have undesirable problems such as damage to various blood cells, elution of harmful components from leukocytes, contamination of bacteria, and the like.

To overcome these problems, if leukocytes can be removed by filtration to afford blood products for transfusion when collecting blood from suppliers, the best blood preparation product can be provided in the both viewpoints of the effective utilization of whole blood and the product safety.

Unfortunately, it has been difficult to obtain a filter material that can selectively remove leukocytes directly from whole blood while allowing erythrocytes, blood plasma, and thrombocytes to easily pass through, because according to conventional technologies, increasing the removal rate of leukocytes accompanies removing thrombocytes having high adhesivity at the same time, in greater or lesser degrees. Therefore, development of a novel blood filter has been strongly desired.

Japanese Patent Application Laid-open No. 1-249063 (Pall Corporation), for example, discloses a blood filter made by grafting hydrophilic monomers onto a filter material to increase the critical wetting surface tension (CWST) of the filter to 90 or more. Japanese Patent Application Publication (koukoku) No. 6-57248 (Japan Medical Supply Co., Ltd.) discloses a filter made by grafting a water soluble nonionic polymer onto a filter material by chemical bonding. Although a comparatively high thrombocyte yield may be expected from a thrombocyte solution with a high thrombocyte concentration using these technologies, their leukocyte-removing performance was not necessarily satisfactory due to the high hydrophilic properties. Therefore, those filters cannot selectively remove only leukocytes from blood components containing the leukocytes and thrombocytes such as whole blood and recover thrombocytes at a high efficiency. Neither teachings nor suggestions are disclosed on the possibility of obtaining a filter material that can selectively remove only leukocytes directly from the whole blood while allowing erythrocytes, blood plasma, and thrombocytes to easily pass through.

In addition, the above methods require special equipments (equipments for radiating radial ray or electron beam) for the grafting reaction, and have more basic problems due to elution and the like accompanying with the degradation of the filter materials when the irradiation time and strength are adjusted to increase the grafting yield. There have been no materials for use in the medical field that are industrially satisfactory in terms of processes, safety, and cost.

DISCLOSURE OF THE INVENTION

An object of the present invention is to provide a leukocyte-removing filter, which has very slight adherence property of thrombocytes , can selectively remove only leukocytes from whole blood or blood components containing leukocytes and thrombocytes, and can recover erythrocytes, blood plasma, and thrombocytes, particularly thrombocytes at a high yield.

The inventor of the present invention has conducted extensive studies on behaviors of various components in whole blood to porous filters, particularly on the leukocyte-removing characteristics and the adhesion- or permeation behavior of thrombocytes during filtration of whole blood. As a result, the inventor has found the surprising fact that a blood filter comprising a polymer containing both hydrophobic and hydrophilic structural units ("component (A)") and a porous substrate ("component (B)") exhibits both high leukocyte-removing capability and thrombocyte recovery capability. This finding has led to the completion of the present invention.

The polymer containing both a hydrophobic structural unit and hydrophilic structural unit in the present invention is a polymer containing one or more hydrophobic structural units represented by the following formulas (1) to (4), for example.

$$-CR^1R^2-CR^3R^4- \quad (1)$$

$$-CR^5=CR^6- \quad (2)$$

$$-C\equiv C- \quad (3)$$

$$-CR^7R^8R^9 \quad (4)$$

wherein $R^1$ to $R^9$ individually represents a hydrogen, halogen atom, alkyl group having 1-12 carbon atoms, aromatic compound having 6-12 carbon atoms, heterocyclic compound having 5-12 carbon atoms or macromer having a number average molecular weight of 500-50,000 and/or an alkyl group having 1-12 carbon atoms, aromatic compound having 6-12 carbon atoms, heterocyclic compound having 5-12 carbon atoms or macromer having a number average molecular weight of 500-50,000, to which functional group selected from carboxylic acid group, carbonyl group, acid anhydride group, carboxylate group, epoxy group, ether group, carbonate group, sulfonic acid, sulfonate group, substituted amide group, isocyanate group, and alkoxysilane group is added, or derivative group thereof is added. The hydrophobic structural unit as used in the present invention is a hydrophobic monomer unit represented by any one of the above formulas (1) to (4). Said structure may be introduced into a polymer chain by any conventionally known method. The conventionally known methods may include a method of copolymerizing a hydrophobic monomer and a hydrophilic monomer, a method of homopolymerizing a macromer containing a hydrophobic structural unit and a hydrophilic structural unit, or copolymerizing a macromer having a hydrophobic structural unit and a hydrophilic monomer or a macromer having a hydrophilic structural unit, a method of grafting a hydrophobic monomer in a part of the homopolymer chain obtained by homopolymerizing hydrophilic monomer, a method of adding a hydrophobic monomer at the terminal of the homopolymer chain obtained by polymerizing a hydrophilic monomer, a method of polymerizing a hydrophilic monomer, then denaturing or chemically modifying (esterification, amidization, alkylation, halogenation, hydrogenation, etc.) a part of the resulting homopolymer chain to introduce a hydrophobic structural unit, and the like. The best method can be selected according to the object, reaction conditions, processes, cost, and the like. In the polymer chain of the polymer of the component (A), the hydrophobic structural units may be present in any arrangement including, but not specifically limited to, a random arrangement, alternation arrangement, block arrangement, graft arrangement, or the like, as required.

When a hydrophobic monomer unit of the formulas (1)-(4) is a structural unit having a crosslinkable functional group, the chemical structures formed after introduction of this structure by the crosslinking reaction of crosslinkable functional groups of two or more monomers also include the hydrophobic structural unit of the present invention. Such a hydrophobic structural unit is one of the most preferable structures in the present invention to cause both high leukocyte-removing capability and high thrombocyte recovery capability to exhibit at the same time.

The hydrophilic structural unit in the present invention is a hydrophilic monomer represented by, for example, the following formula (5),

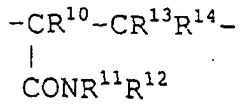

(5)

wherein $R^{10}$ to $R^{14}$ are individually a hydrogen atom or an alkyl group having 1-9 carbon atoms, provided that at least one of the groups $R^{11}$ or $R^{12}$ is an alkyl group.

Although details of the performance expression principle of the present invention are still to be clarified, the hydrophobic structural units of the formulas (1)-(4) in the polymer may effectively act to leukocyte-removing performance on the surface of the porous filter of the present invention due to the hydrophobic interaction and the like, whereas the non-cationic and highly hydrophilic structural unit of the formula (5) may act to suppress thrombocyte adhesion. As a result, it is thought that both compatibility between the high leukocyte-removing capability and efficient thrombocyte recovery capability have been realized.

The present invention will now be described in more detail.

The leukocyte-removing filter in the present invention means a porous filter which can selectively remove only leukocytes from blood components containing leukocytes and thrombocytes such as whole blood and can recover thrombocytes at a high efficiency.

The hydrophobic structural unit in the polymer of the present invention (component (A)) is a structural unit represented by any one of the following formulas (1)-(4) or a derivative thereof. When the hydrophobic structural unit has a crosslinkable functional group, the cross-linking molecular structure after the crosslinking reaction is also within the scope of the hydrophobic structural unit of the present invention.

$$-CR^1R^2-CR^3R^4- \qquad (1)$$

$$-CR^5=CR^6- \qquad (2)$$

$$-CR^5=CR^6- \qquad (2)$$

$$-C\equiv C- \qquad (3)$$

$$-CR^7R^8R^9 \qquad (4)$$

wherein $R^1$ to $R^9$ individually represents a hydrogen, halogen atom, alkyl group having 1-12 carbon atoms, aromatic compound having 6-12 carbon atoms, heterocyclic compound having 5-12 carbon atoms or macromer having a number average molecular weight of 500-50,000 and/or an alkyl group having 1-12 carbon atoms, aromatic compound having 6-12 carbon atoms, heterocyclic compound having 5-12 carbon atoms or macromer having a number average molecular weight of 500-50,000, which is added a functional group selected from carboxylic acid group, carbonyl group, acid anhydride group, carboxylate group, epoxy group, ether group, carbonate group, sulfonic acid, sulfonate group, substituted amide group, isocyanate group, and alkoxysilane group, or a derivative group thereof.

The hydrophobic structural unit of the above formulas (1) to (4) may have hydrophilic groups to the extent that the structural unit is hydrophobic as a whole.

The hydrophobic structural unit in the component (A) of the present invention is hydrophobic monomer unit represented by the formulas (1) - (4) or the derivative thereof, the derivative indicates a reaction product of the hydrophobic structural units , a reaction product of a functional group in the hydrophobic structural unit and other functional groups in the polymer, and the like.

For introducing such a hydrophobic structural unit into the polymer (component (A)), any conventionally known method can be suitably selected and adopted.

Such a conventionally known method includes, for example, a method of copolymerizing a hydrophobic monomer and a hydrophilic monomer, a method of homopolymerizing a macromer containing a hydrophobic structural unit and a hydrophilic structural unit, or copolymerizing a macromer having a hydrophobic structural unit and a hydrophilic monomer or a macromer having a hydrophilic structural unit, a method of grafting a hydrophobic monomer in part of

the homopolymer chain obtained by homopolymerizing a hydrophilic monomer, a method of adding hydrophobic monomer at the terminal of the homopolymer chain obtained by polymerizing hydrophilic monomer, a method of polymerizing hydrophilic monomer, then denaturing or chemically modifying (alkylation, halogenation, hydrogenation, etc.) part of the resulting homopolymer chain to introduce a hydrophobic structural unit, and the like.

As a preferable crosslinkable functional group to be introduced in the polymer (component (A)) of the present invention, an alkoxysilane group and its derivatives, isocyanate group, epoxy group (glycidyl group) , and an acid anhydride group can be given. Among these crosslinkable functional groups, an alkoxysilane group is preferable from the viewpoint of reactivity. More specifically, a trimethoxysilane group and triethoxysilane group are particularly preferable crosslinkable functional groups. One or more crosslinkable functional groups may be introduced in the polymer chain, with no specific limitations to the number.

Examples of hydrophobic monomers for forming the hydrophobic structural units of the formulas (1)-(4) include olefin-type monomers such as ethylene, propylene, 1-butene, cyclopentene, and norbornene; styrene-type monomers such as styrene, $\alpha$-methylstyrene, and divinylbenzene; heterocycle-containing monomers such as N-vinyl pyridine, N-vinyl caprolactam, and N-vinyl valerolactam; diene-type monomers such as butadiene, isoprene, 1,3-cyclohexadiene, 1,3-octadiene, and norbornadiene; ester-type monomers such as methacrylic acid ester and acrylic acid ester; cyclic siloxanes ; and the like. Examples of the hydrophobic monomers having crosslinkable functional groups include $\gamma$-methacryloxypropyl trimethoxysilane, glycidyl methacrylate, and methacryloxypropyl isocyanate.

Of these hydrophobic monomers, ester-type monomers such as methacrylic acid esters and acrylic acid esters (alkyl esters of methacrylate or acrylate) are preferable hydrophobic monomers in the present invention. 2-hydroxy propyl methacrylate, methyl acrylate, methyl methacrylate, ethyl acrylate, ethyl methacrylate, butyl acrylate, butyl methacrylate, 2-ethylhexyl acrylate, and 2-ethylhexyl methacrylate are given as examples of particularly preferable hydrophobic monomers. These monomers may be used either individually or in combination of two or more. As particularly preferable hydrophobic monomers, 2-hydroxypropyl methacrylate, methyl methacrylate, ethyl methacrylate, and butyl methacrylate can be given. The most preferable hydrophobic monomers are 2-hydroxypropyl methacrylate and methyl methacrylate.

When the hydrophobic monomers have a crosslinkable functional group, $\gamma$-methacryloxypropyltrimethoxysilane, $\gamma$-methacryloxypropyltriethoxysilane, $\gamma$-methacryloxypropylmethyldimethoxysilane, 2-methacryloyloxyethylisocyanate, and glycidyl methacrylate are preferable, and particularly preferable monomer is $\gamma$-methacryloxypropyltrimethoxysilane.

As a hydrophilic monomer for forming the hydrophilic structural unit of the formula (5) in the present invention, N,N'-disubstituted acrylamides, N-substituted acrylamides, N,N'-disubstituted methacrylamides, and N-substituted methacrylamides can be given as examples.

As preferable hydrophilic monomers in the present invention, N,N'-disubstituted acrylamides and N,N'-disubstituted methacrylamides can be given, and N,N'-disubstituted acrylamides are particularly preferable as hydrophilic monomers.

As specific examples of preferable hydrophilic monomer in the present invention, N,N'-dimethyl acrylamide, N-methyl acrylamide, N,N'-diethyl acrylamide, N-ethyl acrylamide, N-isopropyl acrylamide, N,N'-diisopropyl acrylamide, N-butyl acrylamide, N,N'-dibutyl acrylamide, N-acryloylmorpholine, N,N'-dimethyl methacrylamide, and N-methyl methacrylamide can be given. Particularly preferable hydrophilic monomers are N,N'-dimethyl acrylamide and N,N'-diethyl acrylamide. From the viewpoint of industrial application, the most preferable hydrophilic monomer is N,N'-dimethyl acrylamide.

Known conventional methods of polymerization can be adopted as are for synthesizing the polymer (component (A)) of the present invention. For example, radical polymerization, ionic polymerization, coordination polymerization, condensation polymerization, and the like can optionally be adopted depending on the purpose to synthesize hydrophilic polymers having desired structures.

The structure of the polymer chain formed by the copolymerization of a hydrophobic monomer (including a monomer having a cross-linking functional group) and a hydrophilic monomer of the present invention may be a random structure, alternation structure, graft structure, or block structure. In addition, a method of synthesizing a macromer by previously polymerizing a hydrophobic monomer and/or a hydrophilic monomer by radical polymerization or ionic polymerization, followed by copolymerization of another hydrophobic monomer and/or a hydrophilic monomer with the macromer may be applied as appropriate.

The number average molecular weight of the polymer (component (A)) of the present invention may be from 1,000 to 1,000,000, preferably from 5,000 to 800,000, and particularly preferably from 20,000 to 500,000. If number average molecular weight is not more than 1,000, the effect of the present invention cannot be sufficiently exhibited and the storage stability over a long period of time may be lost. On the other hand, if the number average molecular weight is not less than 1,000,000, not only is the solubility of the polymer decreased, but also the viscosity of the solution is remarkably increased, giving rise to undesirable effects such as difficulty in blood filter manufacturing.

The ratio of the hydrophobic structural units represented by the formulas (1)-(4) in the polymer of the component (A) of the present invention, that is (the number of the hydrophobic structural units of the formulas (1)-(4))/(the total number of the hydrophilic structural units of the formula (5) and the hydrophobic structural units of the formulas (1)-(4)), is preferably in the range of 0.5-99.5 mol%, more preferably 2-98 mol%, and particularly preferably 5-95 mol%. If the ratio of the hydrophobic structural units is not more than 0.5 mol% or not less than 99.5 mol%, compatibility of high leukocyte-removing effect and efficient thrombocyte recovery performance cannot be realized.

Although the ratio of the hydrophobic structural units in the polymer of the component (A) is preferable in the above range, the polymer (component (A)) of the present invention is preferably a hydrophilic property polymer exhibiting hydrophilic properties as a whole.

To the extent not affecting the characteristics of the leukocyte-removing filter of the present invention, the main chain of the component (A) polymer may include structural unit having at least one bond selected from ether bond, ester bond, amide bond, imide bond, urethane bond, sulfonate bond, and sulfide bond, without any specific limitations.

The porous substrate (component (B)) in the present invention has a number of fine pores continuing one surface to the other surface with a pore diameter capable of separating and removing leukocytes in the coexistence with the component (A). The shape of the pores, continuing conditions thereof, and thickness, material, figure and dimensions of the substrate, and the like of the component (B) may be optionally varied depending on the purpose, but are not specifically limited. For example, the component (B) of the present invention can be used in any optional form, including the form of fiber, film, sheet, disk, cylinder, membrane, particles or the like.

The porous substrate of the component (B) of the present invention must have a suitable pore diameter for efficiently exhibiting the performance as a leukocyte-removing filter. The average pore diameter required for the component (B) to achieve the object of the present invention is preferably in the range from 0.1 to 100 μm, more preferably from 0.5 to 50 μm, and particularly preferably from 1 to 25 μm. If average pore diameter is not within the range of 0.1 to 100 μm, compatibility of high leukocyte-removing effect and efficient thrombocyte recovery performance cannot be realized.

In the present invention, the average pore diameter of the component (B) can be measured and calculated by known conventional methods such as a mercury porosimeter or a permeability measurement method. The specific surface area of the component (B) is also a very important factor in order to efficiently exhibit the leukocyte-removing capability in the present invention. The specific surface area of the component (B) is preferably from 0.1 to 10.0 $m^2/g$, still more preferably from 0.5 to 5.0 $m^2/g$, and particularly preferably from 0.8 to 3.0 $m^2/g$. If the specific surface area is outside the scope of the present invention, no practical blood filter performance can be achieved, resulting in a poor product value. The specific surface area of the component (B) is measured and calculated by a known conventional method using a specific surface area meter such as the BET adsorption measurement or a mercury porosimeter.

There are no specific limitations on the material for the porous substrate (component (B)) of the present invention so far as the above average pore size and specific surface area are satisfied. Specifically, the material for the component (B) may be any one of an organic material, inorganic material, or an organic/inorganic composite material. In addition, such a material may be a natural material, synthetic material, or semi-synthetic material.

More specific examples which can be given include porous substrates made from natural, synthetic, semi-synthetic, or regenerated organic or inorganic fibers, or a composite material of these fibers; porous substrates being as foamed porous substrates (foamed material, sponge, etc.) formed from an organic material or inorganic material, or a composite material of these materials; organic or inorganic, or organic-inorganic composite porous substrates in which fine pores have been formed by elusion, decomposition, sintering, drawing, punching, phase separation, and the like; or porous substrates filled and/or bonded with particles or fine pieces made from an organic material or inorganic material, or a composite material of these materials.

When the component (B) of the present invention is formed from fibers, for example, there are no specific limitations to the properties of the fibers themselves. Organic, inorganic, or composite short fibers, hollow fibers, and long fibers may be appropriately adopted. The form of the component (B) made from fibers may be a simple filled-aggregate of fibers, nonwoven fabric, knitted fabric, or woven fabric so far as the average pore size and specific surface area are within the scope of the present invention.

When the component (B) of the present invention is nonwoven fabric, the fibers forming the nonwoven fabric preferably have diameter of 0.1-10 μm and bulk density of 0.05-1 $g/cm^3$, particularly preferably diameter of 0.5-5 μm and bulk density of 0.1-0.8 $g/cm^3$ to achieve the effect of the present invention.

To selectively remove only leukocytes from blood composition, such as whole blood, containing leukocytes and thrombocytes and recover thrombocytes at a high yield using the leukocyte-removing filter of the present invention, the component (A) must be present as homogeneously as possible in the surface layer including the inside of the porous material of the component (B). Both high leukocyte-removing performance and efficient thrombocyte recovery performance of the present invention can be achieved during filtration of whole blood by the appropriate characteristics of the surface layer for various blood components as well as the appropriate pore size formed from the component (A) and the component (B) . In this instance, the ratio of the component (A) and the component (B) forming the leukocyte-removing filter of the present invention is also one of very important factor to maximize the effect of the present invention.

The ratio by weight of the polymer (component (A)) to the porous substrate (component (B)) of the present invention is preferably 0.001-1.0, and more preferably 0.001-0.8, and most preferably 0.01-0.5. If the ratio by weight of the component (A) to the component (B) is not less than 1.0, the component (A) plugs pores of the component (B), that brings about undesirable results such as impaired performance as a porous substrate, or an increased cost of the product in the point of industrial view. On the contrary, if the ratio by weight of the component (A) to the component (B) is not more than 0.001, there may exist the possibility forming some parts that the component (A) is not present in the surface layer of the component (B). Such a filter may not sufficiently exhibit the effect of the leukocyte-removing filter of the present invention.

As a preferable method for manufacturing the leukocyte-removing filter of the present invention exhibiting both high leukocyte-removing capability and thrombocyte recovery capability, a method of coating the hydrophilic polymer (component (A)) onto the porous substrate (component (B)) (including the surface layer inside the porous material) can be given. Such a method comprises preparing a solution contained the polymer having hydrophobic structural unit (component (A)) (inorganic solvent, aqueous solution, ormixture of these) or putting component (A) into fluid condition by any suitable means, then applying component (A) given fluidity to a porous substrate of the component (B) by immersion or spraying, or filling the porous substrate with the component (A) under pressure; or applying fine particles of the component (A) onto the component (B), and then heating the material to dissolve the component (A). The combination of two or more methods above may be acceptable without any specific limitations. Conventional methods for modifying the surface of blood filters require very expensive and complicated apparatuses and procedures such as irradiation of electron beams or gamma rays. According to the methods of the present invention, however, the filter can exhibit excellent leukocyte reduction performance by simply causing the component (A) to be present on the surface layer of the component (B) by coating, which is industrially a very advantageous method.

In the manufacturing method of the present invention a drying step such as air-blowing, rolling, and heating may be combined after coating the component (A) onto the component B (the surface layer: including inside of the porous material).

When the hydrophobic structural unit of the component (A) of the present invention has crosslinkable functional group, the crosslinking reaction of the crosslinkable functional group may be carried out during the coating process, drying process, or heating process of the present invention. The leukocyte-removing filter having the crosslinking structure obtained by such a production process can exhibit a particularly preferred performance in the present invention.

Among the blood filters obtained by the manufacturing method of the present invention, the filters prepared by coating the component (A), which contains both the hydrophobic structural unit and the hydrophobic structural unit having a crosslinkable functional group, onto the component (B), followed by the crosslinking reaction to form a stable surface layer of the component (A) onto the component (B), can exhibit both excellent leukocyte-removing performance and high thrombocyte recovering performance at the same time, can also exhibit further superior performances such as endurance to sterilization, resistance to autoclave (heat resistance) , long-term stability, and the like. Such a filter is the most preferable product of the present invention.

Although some possibilities can be suggested about the performance expression mechanism of the novel leukocyte-removing filter of the present invention, the most important factor would be the presence of the component (A) on the surface of the porous substrate (component (B)) where the filter comes into contact with various blood components. Furthermore, the hydrophilic properties on the surface of the polymer (component (A)) may be slightly hydrophobicized by the introduction of hydrophobic structural units into the component (A), whereby, although the surface remains to be hydrophilic, the molecular characteristics advantageous to leukocyte capturing such as hydrophobic interaction properties may be acquired. On the other hand, the introduction of the hydrophilic structural units into the component (A) reduces the interaction of the surface characteristic of the polymer (component (A)) with proteins in plasma and suppresses adsorption of plasma proteins, which activates thrombocytes, onto the filter surface. This may contribute to recovery of thrombocytes from whole blood at high efficiency. Of course, an appropriate pore diameter plays an important role in the efficient removal of leukocytes from whole blood.

The novel leukocyte-removing filter of the present invention can be effectively used for whole blood, and also can be applied to selective removal of leukocytes from various blood products and thrombocyte suspensions in which leukocytes are present without specific limitation. For example, the filter may be used for removing leukocytes from a concentrated thrombocyte solution obtained by centrifugation of whole blood immediately after collection. In addition, the leukocyte-removing filter of the present invention may be used in combination with a pre-filter for removing fine coagulated components when filtering whole blood or thrombocyte suspension containing leukocytes. The leukocyte-removing filter of the present invention may be-independently filled in a filter housing or may be used incorporating into a bag apparatus for blood component separation aseptically connected a blood collecting bag and a bag for blood component separation.

PREFERRED EMBODIMENT OF THE INVENTION

The present invention will be explained in more detail by examples and comparative examples which are not intended to limit the present invention.

Examples

Synthesis Example 1

Synthesis of copolymer of N,N'-dimethylacrylamide (DMAA) and butylmethacrylate (BMA)

A 1 litter separable flask equipped with a stirrer and a nitrogen feed pipe was charged with 69.4 g (0.7 mol) of DMAA and 42.8 g (0.3 mol) of BMA. 200 ml of ethanol (EtOH) was then added to dissolve the mixture. Nitrogen gas was injected into the mixed solution for 3 minutes to replace the reaction system with nitrogen. The solution was stirred at 60°C under the nitrogen atmosphere. Solution of 0.8 g (0.05 mol) of 2,2-azobisisobutyronitrile (AIBN) in 100 ml of EtOH was continuously dripped over three hours as a polymerization initiator. The polymerization reaction was carried out for further two hours after the completion of dripping. After completion of the reaction, the reaction solution was cooled to 25°C and poured into a large amount of water to precipitate the polymer. The polymer was collected from the reaction mixture by filtration, washed with n-hexane, and dried. The polymer obtained was confirmed to be copolymer with DMAA:BMA ratio of 56.4:43.6 (mol% ratio) (calculated from the result of $^1$H-NMR measurement), number average molecular weight (Mn) of 56,300 and weight average molecular weight (Mw) of 129,000 (calculated from the result of GPC measurement using the standard MMA).

Synthesis Example 2

Synthesis of copolymer of DMAA and methyl methacrylate (MMA)

The polymerization reaction and analysis of the resulting polymer were carried out in the same manner as in Synthesis Example 1, except for using 54.6 g (0.55 mol) of DMAA and 45.0 g (0.45 mol) of MMA as monomers. The resulting polymer was confirmed to have a DMAA:MMA ratio of 38.8:61.2 (mol% ratio), Mn of 68,900, and Mw of 137,000.

Synthesis Example 3

Synthesis of copolymer of DMAA and 2-hydroxypropyl methacrylate (HPMA)

The polymerization reaction was carried out in the same manner as in Synthesis Example 1, except for using 49.6 g (0.50 mol) of DMAA and 72.1 g (0.50 mol) of HPMA as monomers. After completion of the reaction, the reaction solution was cooled to 25°C and poured into a large amount of n-hexane to precipitate the polymer. The polymer was collected from the reaction mixture by filtration, washed with purified water, and dried. The resulting polymer was confirmed to be copolymer with DMAA:HPMA ratio of 36.8:63.2 (mol% ratio), Mn of 139,000, and Mw of 404,000 (the polymer was analyzed in the same manner as in Synthesis Example 1).

Synthesis Example 4

Synthesis of copolymer of DMAA and γ-methacryloxypropyltrimethoxysilane (MPTS)

The polymerization reaction was carried out in the same manner as in Synthesis Example 1, except for using 94.2 g (0.95 mol) of DMAA and 12.4 g (0.05 mol) of MPTS as monomers. After completion of the reaction, the reaction solution was cooled to 25°C and poured into a large amount of n-hexane to precipitate the polymer. The polymer was collected from the reaction mixture by filtration, washed with n-hexane, and dried. The resulting polymer was confirmed to be copolymer having a DMAA:MPTS ratio of 95.9:4.1 (mol% ratio), Mn of 17,500, and Mw of 76,200.

Synthesis Example 5

Synthesis of ternary copolymer of DMAA, MPTS, and MMA

The polymerization reaction and the analysis of the polymer were carried out in the same manner as in Synthesis Example 4, except for using 69.1 g (0.7 mol) of DMAA, 12.4 g (0.05 mol) of MPTS, and 25.3 g (0.25 mol) of MMA as monomers. The resulting polymer was confirmed to be copolymer having a DMAA:MPTS:MMA ratio of 62.8: 3.9:33.3 (mol% ratio), Mn of 42,600, and Mw of 118,000.

Synthesis Example 6

Synthesis of copolymer of N-acryloylmorpholine (ACMO) and BMA

The polymerization reaction was carried out in the same manner as in Synthesis Example 1, except for using 120 g (0.85 mol) of ACMO and 21.3 g (0.15 mol) of BMA as monomers, and N,N-dimethylformamide (DMF) as polymerization solvent. After completion of the reaction, the reaction solution was cooled to 25°C and poured into a large amount of EtOH to precipitate the polymer. The polymer was collected from the reaction mixture by filtration, washed with water, and dried. The resulting polymer was confirmed to be copolymer with ACMO:BMA ratio of 83.9:16.1 (mol% ratio), Mn of 37,900, and Mw of 99,400 (the polymer was analyzed in the same manner as in Synthesis Example 1).

Synthesis Example 7

Synthesis of copolymer of ACMO and MMA

The polymerization reaction and the analysis of the polymer were carried out in the same manner as in Synthesis Example 6, except for using 98.8 g (0.7 mol) of ACMO and 30.0 g (0.3 mol) of MMA as monomers.
The resulting polymer was confirmed to be copolymer having ACMO:MMA ratio of 58.4:41.6 (mol% ratio), Mn of 72,500, and Mw of 159,000.

Synthesis Example 8

Synthesis of copolymer of ACMO and MPTS

The polymerization reaction was carried out in the same manner as in Synthesis Example 6, except for using 134.6 g (0.95 mol) of ACMO and 12.4 g (0.05 mol) of MPTS as monomers. After completion of the reaction, the reaction solution was cooled to 25°C and poured into a large amount of n-hexane to precipitate the polymer. The polymer was collected from the reaction mixture by filtration, washed with purified water, and dried. The resulting polymer was confirmed to be copolymer with ACMO:MPTS ratio of 95.2:4.8 (mol% ratio) , Mn of 73,100, and Mw of 159,000 (the polymer was analyzed in the same manner as in Synthesis Example 1).

Synthesis Example 9

Synthesis of copolymer of DMAA and MMA macromer

The polymerization reaction and analysis of the resulting polymer were carried out in the same manner as in Synthesis Example 1, except for using 79.3 g (0.8 mol) of DMAA and 20.0 g of a commercially available MMA macromer (0.2 mol% as MMA) as monomers, and DMF as a reaction solvent. The resulting polymer was confirmed to be copolymer having ratio of DMAA:MMA in macromer of 77.0:23.0 (mol% ratio), Mn of 31,000, and Mw of 87,000.

Synthesis Example 10

Synthesis of polymer with a hydrophobic structural unit content of 99.5 mol% or more

The polymerization reaction and analysis of the polymer were carried out in the same manner as in Synthesis Example 1, except for using 0.594 g (0.006 mol) of DMAA and 141.3 g (0.994 mol) of BMA as monomers. The resulting polymer was confirmed to be copolymer having DMAA:BMA ratio of 0.4:99.6 (mol% ratio), Mn of 86,000, and Mw of 179,000.

Synthesis Example 11

Synthesis of polymer with a hydrophobic structural unit content of less than 0.5% (1)

The polymerization reaction and analysis of the polymer were carried out-in the same manner as in Synthesis

Example 1, except for using 98.9 g (0.998 mol) of DMAA and 0.284 g (0.002 mol) of BMA as monomers. The resulting polymer was confirmed to be copolymer having DMAA:BMA ratio of 99.6/0.4 (mol% ratio), Mn of 29,500, and Mw of 78,200.

Synthesis Example 12

Synthesis of polymer with a hydrophobic structural unit content of less than 0.5% (2)

The polymerization reaction and analysis of the polymer were carried out in the same manner as in Synthesis Example 4, except for using 98.7 g (0.996 mol) of DMAA and 0.994 g (0.004 mol) of MPTS as monomers. The resulting polymer was confirmed to be copolymer having DMAA:MPTS ratio of 99.7:0.3 (mol% ratio), Mn of 21,800, and Mw of 74,200.

The results of Synthesis Examples 1-12 are summarized in Table 1.

Table 1

| Polymers of Synthesis Examples | | | | |
|---|---|---|---|---|
| | Monomers (mol% ratio) | Polymers (mol% ratio) | Mn | Mw |
| Synthesis Example 1 | DMAA/BMA 70.0/30.0 | DMAA/BMA 56.4/43.6 | 56300 | 129000 |
| Synthesis Example 2 | DMAA/MMA 55.0/45.0 | DMAA/MMA 38.8/61.2 | 68900 | 137000 |
| Synthesis Example 3 | DMAA/HPMA 50.0/50.0 | DMAA/HPMA 36.8/63.2 | 139000 | 404000 |
| Synthesis Example 4 | DMAA/MPTS 95.0/5.0 | DMAA/MPTS 95.9/4.1 | 17500 | 76200 |
| Synthesis Example 5 | DMAA/MPTS/MMA 70.0/5.0/25.0 | DMAA/MPTS/MMA 62.8/3.9/33.3 | 42600 | 118000 |
| Synthesis Example 6 | ACMO/BMA 85.0/15.0 | ACMO/BMA 83.9/16.1 | 37900 | 99400 |
| Synthesis Example 7 | ACMO/MMA 70.0/30.0 | ACMO/MMA 58.4/41.6 | 72500 | 159000 |
| Synthesis Example 8 | ACMO/MPTS 95.0/5.0 | ACMO/MPTS 95.2/4.8 | 73100 | 159000 |
| Synthesis Example 9 | DMAA/MMA (Converted to MMA in macromer) 80.0/20.0 | DMAA/MMA (Converted to MMA in macromer) 77.0/23.0 | 31000 | 87000 |
| Synthesis Example 10 | DMAA/BMA 0.6/99.4 | DMAA/BMA 0.4/99.6 | 86000 | 179000 |
| Synthesis Example 11 | DMAA/BMA 99.8/0.2 | DMAA/BMA 99.6/0.4 | 29500 | 78200 |
| Synthesis Example 12 | DMAA/MPTS 99.6/0.4 | DMAA/MPTS 99.7/0.3 | 21800 | 74200 |

Preparation Examples

Preparation Example 1

A nonwoven fabric made from polyethylene terephthalate (PET) with a size of 15 cm × 20 cm (average fiber diameter: about 1.2 μm, nicking ("Metsuke") : about 40 g/m$^2$, thickness: 190 μm) was dipped in 200 ml of EtOH solution of the polymer obtained in the Synthesis Example 1 (polymer concentration: 5 wt%) for 3 minutes. After removing excessive polymer solution by squeezing the fabric between nip rollers, the fabric was dried under vacuum for 5 hours at 40°C to obtain a blood filter (A) coated with 16.3 wt% of polymer, wherein the coated amount (wt%) = (the weight nonwoven fabric after coating (g) - the weight nonwoven fabric before coating (g) ) / (the weight nonwoven fabric before coating (g)) x 100, or (the weight of component (A)/the weight of component (B)) x 100.

Preparation Examples 2-3

Blood filters (B) and (C), coated with polymer respectively in the coated amount of 22.7 wt% and 20.7 wt%, were prepared in the same manner as in the Preparation Example 1, except for using the polymers obtained in the Synthetic Examples 2 and 3, respectively.

Preparation Example 4

The polymer obtained in the Synthesis Example 4 was dissolved in mixed solution of water and ethanol (volume ratio, water:EtOH = 2:8), to which dilute hydrochloric acid was added to make an HCl concentration of 0.002 mol/l, to prepare solution with polymer concentration of 5 wt%. The nonwoven fabric used in the Preparation Example 1 was dipped in 200 ml of this polymer solution for 3 minutes. After removing excessive polymer solution by squeezing the fabric between nip rolls, the fabric was dried with heating for one hour at 80°C to obtain a blood filter (D) coated with 24.0 wt% of the polymer.

Preparation Example 5

A blood filter (E) coated with 21.6 wt% of polymer was prepared in the same manner as in the Preparation Example 4, except for using the polymer obtained in the Synthetic Example 5.

Preparation Example 6

The nonwoven fabric used in the Preparation Example 1 was dipped in 200 ml of a dioxane solution of the polymer obtained in the Synthesis Example 6 (polymer concentration: 5 wt%) for 3 minutes. After removing excessive polymer solution by squeezing the fabric between nip rolls, the fabric was dried under vacuum for 5 hours at 40°C to obtain a blood filter (F) coated with 22.0 wt% of the polymer.

Preparation Example 7

A blood filter (G) coated with 20.0 wt% of polymer was prepared in the same manner as in the Preparation Example 6, except for using the polymer obtained in the Synthetic Example 7.

Preparation Example 8

The polymer obtained in the Synthesis Example 8 was dissolved in mixed solution of water and dioxane (water:dioxane = 2:8, volume ratio) , to which dilute hydrochloric acid was added to make an HCl concentration of 0.002 mol/l in advance, to obtain a solution with polymer concentration of 5 wt%. The nonwoven fabric was dipped 200 ml of this polymer solution for 3 minutes. After removing excessive polymer solution by squeezing the fabric between nip rolls, the fabric was dried with heating for one hour at 80°C to obtain a blood filter (H) coated with 15.0 wt% of the polymer.

Preparation Example 9

A blood filter (I) coated with 17.0 wt% of polymer was prepared in the same manner as in the Preparation Example 1, except for using the polymer obtained in the Synthetic Example 9.

Preparation Examples 10-11

Blood filters (J) and (K), coated with polymer respectively in the amount of 18.0 wt% and 21.0 wt%, were prepared in the same manner as in the Preparation Example 1, except for using the polymers obtained in the Synthetic Examples 10 and 11, respectively.

Preparation Example 12

A blood filter (L) coated with 19.0 wt% of polymer was prepared in the same manner as in the Preparation Example 4, except for using the polymer obtained in the Synthetic Example 12.

Preparation Example 13

Preparation of blood filter by grafting method

The nonwoven fabric used in the Preparation Example 1 was dipped in 5 wt% DMAA ethanol solution in a 51 flask. Nitrogen gas was injected into the solution for 3 minutes to replace the reaction system with nitrogen. γ-rays at a dose of 3.6 kGy (1.2 kGy/hour) were applied to the nonwoven fabric to cause the monomer to polymerize on the

fabric surface by graft polymerization. After the reaction, the nonwoven fabric was taken out, washed repeatedly with purified water, and dried under vacuum for 5 hours at 40°C. The blood filter (M) obtained was confirmed to have DMAA polymer graft ratio of 9.0 wt% (graft ratio = (weight after grafting/weight before grafting-1) x 100 (%)).

Preparation Example 14

A blood filter (N) coated with 0.04 wt% of polymer was prepared in the same manner as in the Preparation Example 1, except for using an EtOH solution of the polymer (0.02 wt%) obtained in the Synthetic Example 2.

Preparation Example 15

A blood filter (0) coated with 102.0 wt% of polymer was prepared in the same manner as in the Preparation Example 1, except for using an EtOH solution of the polymer (60 wt%) obtained. in the Synthetic Example 2.

Blood evaluation method

The following two evaluation methods were used to evaluate performance of the blood filters prepared in the Preparation Examples.

Blood evaluation method (1)

The nonwoven fabrics coated with polymer prepared in Preparation Examples were cut into disks with a diameter of 25 mm. Four disks were laminated and filled in a Teflon column. The fresh whole blood used in the all blood evaluations including the description below indicates the whole blood prepared by feeding 100 ml of collected blood into a blood bag containing 14 ml of a CPD solution (composition: 26.3 g/l of sodium citrate, 3.27 g/l of citric acid, 23.2 g/l of glucose, and 2.51 g/l of sodium dihydrogenphosphate dihydrate) as an anti-coagulant, and stored at 20°C for 2 hours after collection. Fresh human whole blood was fed through the column using a syringe pump at a rate of 2.7 ml/min at room temperature to collect 6 ml of filtrate. The filter performance was represented by the leukocyte reduction capability (-Log Reduction) and thrombocyte recovery rate (%). Specifically, after measuring leukocyte concentration and thrombocyte concentration before and after filtration, the leukocyte reduction capability and thrombocytes recovery rate were determined according to the following equation.

$$\text{Leukopheresis capability} = -\text{Log}\ (B\text{-}A)$$

$$\text{Thrombocyte recovery rate} = (D/C) \times 100(\%)$$

Wherein, (A) is a leukocyte concentration before filtration, (B) is a leukocyte concentration after filtration, (C) is a thrombocyte concentration before filtration, and (D) is thrombocyte concentration after filtration.

The leukocyte concentration before filtration was measured by the Turk method by feeding a 10-fold dilution to a Burker-Turk type blood cell counting chamber and counting the number of leukocytes which are present in eight large compartments through an optical microscope. The following Nageotte method was used for the measurement of the leukocyte concentration after filtration. Specifically, 1ml of the blood after filtration was diluted to 10-foldwithLeucoplate (SOBIODA) and allowed to stand for 20-30 minutes at room temperature. Leukocytes were precipitated by centrifugation. The supernatant liquid containing the other blood components was removed and again adjusted by the leucoplate to 1 ml (non-diluted magnification) , which was added to the Nageotte counting chamber to count the number of leukocytes using an optical microscope. The thrombocyte concentration was measured using an automatic blood cell counter (Sysmex K4500 manufactured by Toa Medical Co., Ltd.). Hematocrit was measured using a hematocrit reader after the blood was put into a glass capillary tube for testing a small quantity blood and centrifuged.

Blood evaluation method (2)

Nonwoven fabrics coated with polymer prepared in the Preparation Examples , or uncoated nonwoven fabrics were cut into disks with a diameter of 20 mm. 32 sheets of the polymer coated nonwoven fabric disks coated (for Examples) or 32 sheets of the uncoated fabric disks (for Comparative Example 7) were layered and filled in a Teflon column, respectively. Fresh whole blood of human was caused to flow at a consistent flow rate of 0.74 ml/min using a syringe pump to recover 13.3 ml of filtrate. The filter performance was represented by the leukocyte reduction capability

(legalistic reduction) and thrombocyte recovery rate (%). Specifically, leukocyte concentration and thrombocyte concentration before and after filtration was measured, and leukocyte concentration before filtration, leukocyte concentration after filtration, thrombocyte concentration before filtration, and thrombocyte concentration after filtration was assumed as (A) , (B) , (C), and (D) , respectively, and the leukocyte reduction capability and thrombocytes recovery rate were determined according to the following formulas :

$$\text{Leukopheresis capability} = -\text{Log (B-A)}$$

$$\text{Thrombocyte recovery rate} = (D/C) \times 100\ (\%)$$

The leukocyte concentration of the fluid before filtration was measured using Leuco COUNT™ kit (BD Bioscience, U.S.) as a residual leukocyte measurement reagent system, of FACS Caliber (BD Bioscience, U.S.) as a flow cytometer, and CELL Quest (BD Bioscience, U.S.) as analytical software. The thrombocyte concentration was measured using MAXMA/L-Retic (BECKMAN COULTER, U.S.) as an automatic blood cell counter.

EXAMPLES

Example 1

The blood evaluation was carried out using the polymer coated nonwoven fabric A prepared in the Preparation Example 1 according to the evaluation methods (1) and (2). The results are shown in Table 2.

Examples 2-5

The blood evaluation was carried out using the polymer coated nonwoven fabrics B to E prepared in the Preparation Examples 2-5 according to the evaluation method (2). The results are shown in Table 2.

Example 6

The blood evaluation was carried out using the polymer coated nonwoven fabric F prepared in the Preparation Example 6 according to the evaluation method (1). The results are shown in Table 2.

Examples 7-9

The blood evaluation was carried out using the polymer coated nonwoven fabrics G to I prepared in the Preparation Examples 7-9 according to the evaluation method (2). The results are shown in Table 2.

Comparative Examples 1-3

The blood evaluation was carried out using the polymer coated nonwoven fabrics J, K, and L prepared in the Preparation Examples 10-12 according to the evaluation method (2). The results are shown in Table 2.

Comparative Example 4

The blood evaluation was carried out using the polymer grafted nonwoven fabric M prepared in the Preparation Example 13 according to the evaluation method (2). The results are shown in Table 2.

Comparative Examples 5-6

The blood evaluation was carried out using the polymer coated nonwoven fabrics N and O prepared in the Preparation Examples 14 and 15 according to the evaluation method (2). The results are shown in Table 2.

Comparative Example 7

The blood evaluation was carried out using an uncoated nonwoven fabric P (average fiber diameter: about 1.2 µm, nicking: about 40 g/$m^2$, thickness: 190 µm) that was used in the Preparation Examples according to the evaluation method (2). The results are shown in Table 2.

Table 2 Blood evaluation results of Examples and Comparative Examples

| | Filter No | Evaluation method | Polymer Composition | Coat amount or graft amount (%) | Leukapheretic rate (-logRed..) | Thrombocytes collect rate (%) |
|---|---|---|---|---|---|---|
| Example 1 | A | (1) | DMAA/BMA 56.4/43.6 | 16.3 | 1.4 | 78.0 |
| | A | (2) | DMAA/BMA 56.4/43.6 | 16.3 | 3.9 | 99.0 |
| Example 2 | B | (2) | DMAA/MMA 38.8/61.2 | 22.7 | 3.0 | 94.2 |
| Example 3 | C | (2) | DMAA/HPMA 36.8/63.2 | 20.7 | 3.7 | 98.1 |
| Example 4 | D | (2) | DMAA/MPTS 95.9/4.1 | 24.0 | 3.4 | 90.0 |
| Example 5 | E | (2) | DMAA/MPTS/MMA 62.8/3.9/33.3 | 21.6 | 3.1 | 88.4 |
| Example 6 | F | (1) | ACMO/BMA 83.9/16.1 | 22.0 | 1.2 | 84.6 |
| Example 7 | G | (2) | ACMO/MMA 58.4/41.6 | 20.0 | 2.0 | 90.0 |
| Example 8 | H | (2) | ACMO/MPTS 95.2/4.8 | 15.0 | 3.3 | 95.0 |
| Example 9 | I | (2) | DMAA/MMA (Converted to MMA in macromer) 77.0/23.0 | 17.0 | 2.0 | 80.0 |
| Comparative Example 1 | J | (2) | DMAA/BMA 0.4/99.6 | 18.0 | 2.5 | 5.0 |
| Comparative Example 2 | K | (2) | DMAA/BMA 99.6/0.4 | 21.0 | 1.0 | 55.0 |
| Comparative Example 3 | L | (2) | DMAA/MPTS 99.7/0.3 | 19.0 | 1.0 | 45.0 |
| Comparative Example 4 | M | (2) | Grafted DMAA | 9.0 (graft amount) | 0.8 | 60.0 |
| Comparative Example 5 | N | (2) | DMAA/MMA 38.8/61.2 | 0.04 | 2.5 | 0.3 |
| Comparative Example 6 | O | (2) | DMAA/MMA 38.8/61.2 | 102 | 2.1 | 15.0 |
| Comparative Example 7 | P | (2) | Uncoated | 0.0 | 3.1 | 0.2 |

INDUSTRIAL APPLICABILITY

According to the present invention, material for the filter that allows erythrocytes, thrombocytes, and blood plasma in a leukocyte-containing fluid represented by whole blood to filter out and selectively and efficiently remove only leukocytes can be provided.

## Claims

1. A leukocyte-removing filter comprising a polymer having a hydrophobic structural unit and a hydrophilic structural unit, and a porous substrate.

2. The leukocyte-removing filter according to claim 1, wherein the polymer has the hydrophobic structural unit and hydrophilic structural unit in the polymer chain.

3. The leukocyte-removing filter according to claim 1 or 2, wherein the polymer is a copolymer of a hydrophobic monomer and hydrophilic monomer.

4. The leukocyte-removing filter according to claim 1, wherein the polymer has the hydrophobic structural unit that has been introduced by denaturing or chemical modification.

5. The leukocyte-removing filter according to any one of claims 1-4, wherein the hydrophobic structural unit in the polymer is incorporated into the polymer chain in a random structure, alternation structure, graft structure, or block structure.

6. The leukocyte-removing filter according to any one of claims 1-5, wherein the hydrophobic structural unit contained in polymer is at least one hydrophobic monomer unit represented by any one of the following formulas (1)-(4) or derivative thereof,

$$-CR^1R^2-CR^3R^4- \quad (1)$$

$$-CR^5=CR^6- \quad (2)$$

$$-C\equiv C- \quad (3)$$

$$-CR^7R^8R^9 \quad (4)$$

wherein $R^1$ to $R^9$ individually represents a hydrogen, halogen atom, alkyl group having 1-12 carbon atoms, aromatic compound having 6-12 carbon atoms, heterocyclic compound having 5-12 carbon atoms or macromer having a number average molecular weight of 500-50,000 and/or alkyl group having 1-12 carbon atoms, aromatic compound having 6-12 carbon atoms, heterocyclic compound having 5-12 carbon atoms or macromer having a number average molecular weight of 500-50,000 which is added a functional group selected from carboxylic acid group, carbonyl group, acid anhydride group, carboxylate group, epoxy group, ether group, carbonate group, sulfonic acid, sulfonate group, substituted amide group, isocyanate group, and alkoxysilane group, or a derivative group thereof..

7. The leukocyte-removing filter according to any one of claims 1-6, wherein the hydrophobic structural unit in the polymer has at least one hydrophobic monomer unit selected from 2-hydroxypropyl methacrylate, methyl methacrylate, and butyl methacrylate or structural unit derived from these hydrophobic monomers.

8. The leukocyte-removing filter according to any one of claims 1-6, wherein at least one hydrophobic structural unit in the polymer has at least one crosslinkable functional group selected from alkoxysilane group, epoxy group, acid anhydride group, and isocyanate group.

**9.** The leukocyte-removing filter according to claim 8, wherein the crosslinkable functional group is an alkoxysilane group or an epoxy group.

**10.** The leukocyte-removing filter according to any one of claims 1-9, wherein the hydrophilic structural unit in the polymer is at least one hydrophilic monomer unit represented by the following formula (5) or derivative thereof,

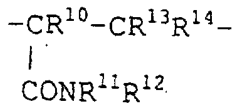

(5)

wherein $R^{10}$ to $R^{14}$ are individually a hydrogen atom or an alkyl group having 1-9 carbon atoms, provided that at least one of the groups $R^{11}$ or $R^{12}$ is an alkyl group.

**11.** The leukocyte-removing filter according to any one of claims 1-10, wherein the hydrophilic monomer unit is an N, N'-disubstituted acrylamide or N-substituted acrylamide, or a structural unit derived from these amides.

**12.** The leukocyte-removing filter according to claim 11, wherein the hydrophilic monomer unit is a dimethylacrylamide.

**13.** The leukocyte-removing filter according to any one of claims 1-12, wherein the polymer comprises a hydrophobic monomer unit represented by any one of the formulas (1)-(4) or a derivative thereof and a hydrophilic monomer unit represented by the formula (5) or a derivative thereof.

**14.** The leukocyte-removing filter according to any one of claims 1-13, wherein ratio of the number of the hydrophobic structural units to the total number of the hydrophilic structural units and the hydrophobic structural units is within the range of 0.5-99.5 mol%, and the polymer contains at least one hydrophobic structural unit.

**15.** The leukocyte-removing filter according to any one of claims 1-14, wherein the porous substrate has pores with average pore size of 0.1-100 μm.

**16.** The leukocyte-removing filter according to any one of claims 1-15 , wherein the porous substrate has specific surface area of 0.1-10.0 $m^2/g$.

**17.** The leukocyte-removing filter according to any one of claims 1-16, wherein the weight ratio of the polymer to the porous substrate is in the range of 0.001-1.0.

**18.** The leukocyte-removing filter according to any one of claims 1-17, being obtainable by coating the porous substrate with the polymer.

**19.** The leukocyte-removing filter according to any one of claims 1-18, being obtainable by coating the porous substrate with the polymer, and optionally crosslinking a part or all of polymer components.

# INTERNATIONAL SEARCH REPORT

International application No.
PCT/JP01/01880

A. CLASSIFICATION OF SUBJECT MATTER
Int.Cl7 A61M 1/34, B01D 39/16

According to International Patent Classification (IPC) or to both national classification and IPC

B. FIELDS SEARCHED

Minimum documentation searched (classification system followed by classification symbols)
Int.Cl7 A61M 1/00-34, B01D 39/00-16

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2001 |
| Kokai Jitsuyo Shinan Koho | 1971-2001 | Jitsuyo Shinan Toroku Koho | 1996-2001 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP, 2000-51623, A (Terumo Corporation), 22 February, 2000 (22.02.00), Full text (Family: none) | 1-13<br>14-19 |
| X<br>Y | JP, 10-33668, A (Asahi Mdical Co., Ltd.), 10 February, 1998 (10.02.98), Full text (Family: none) | 1-13<br>14-19 |
| A | JP, 6-23042, A (Toyobo Co., Ltd.), 01 February, 1994 (01.02.94), Full text (Family: none) | 1-19 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>04 June, 2001 (04.06.01) | Date of mailing of the international search report<br>19 June, 2001 (19.06.01) |
| Name and mailing address of the ISA/<br>Japanese Patent Office<br>Facsimile No. | Authorized officer<br>Telephone No. |

Form PCT/ISA/210 (second sheet) (July 1992)